Europäisches Patentamt

(19) European Patent Office

Office européen des brevets

(11) Numéro de publication : **0 071 495 B1**

(12) **FASCICULE DE BREVET EUROPÉEN**

(45) Date de publication du fascicule du brevet :
**30.09.87**

(21) Numéro de dépôt : **82401202.5**

(22) Date de dépôt : **29.06.82**

(51) Int. Cl.⁴ : **A 61 N   1/05**

(54) **Sonde coaxiale coulissante pour stimulateur cardiaque.**

(30) Priorité : 31.07.81 FR 8114944

(43) Date de publication de la demande :
09.02.83 Bulletin 83/06

(45) Mention de la délivrance du brevet :
30.09.87 Bulletin 87/40

(84) Etats contractants désignés :
AT BE CH DE GB IT LI LU NL SE

(56) Documents cités :
EP-A- 0 009 732
FR-A- 2 446 001
US-A- 3 865 118
US-A- 3 949 757
US-A- 4 332 259

(73) Titulaire : CARDIOFRANCE- COMPAGNIE FRAN-
CAISE D'ELECTROCARDIOLOGIE
40, Grande Rue
F-95450 Commeny (FR)

(72) Inventeur : Buffet, Jacques
28, avenue Thiers
F-93340 Le Raincy (FR)

(74) Mandataire : Derambure, Christian
Cabinet BUGNION ASSOCIES SARL 116, boulevard
Haussmann
F-75008 Paris (FR)

## Description

L'invention concerne une sonde destinée à être associée notamment d'une part à un stimulateur cardiaque implanté et d'autre part au muscle cardiaque.

On connaît déjà un système de deux sondes distinctes associées à une première extrémité à un stimulateur cardiaque, et, par une extrémité opposée, respectivement à l'oreillette et au ventricule. De tels dispositifs sont notamment, mais non exclusivement, utilisés pour un stimulateur synchrone à l'oreillette. L'activité de l'oreillette est décelée au moyen d'une première sonde et la stimulation du ventricule est réalisée au moyen de la seconde sonde.

On connaît aussi, des stimulateurs du type bicavitaire, auquel on associe deux sondes, l'une étant fixée par son extrémité à l'oreillette et l'autre étant fixée par son extrémité au ventricule.

En général, l'accrochage de la sonde au ventricule ne pose pas de problème majeur. Par contre, l'accrochage sur l'oreillette est difficile et son implantation est malaisée. Ainsi, on utilise très rarement à l'heure actuelle des stimulateurs synchrones à l'oreillette, bien que ceux-ci fournissent au malade une stimulation au ventricule adaptée au rythme de l'oreillette et, qu'ils soient bien adaptés pour des sujets jeunes.

On connaît aussi une sonde unique coaxiale, non coulissante comportant deux connexions d'extrémité fixes l'une par rapport à l'autre, destinées à être associées respectivement à l'oreillette et au ventricule. Ainsi, lorsque l'on possède une sonde standard du type connu, il est très difficile de l'adapter à différents malades.

Les brevets américains N° 3 865 118, 3 949 757 et européen N° 9 732 décrivent une sonde comportant deux conducteurs associés de façon à permettre l'ajustement axial de la distance entre la connexion à l'oreillette et celle au ventricule. Il est également connu de ces documents de prévoir des moyens de blocage unidirectionnels d'un conducteur par rapport à l'autre.

Par ailleurs, la demande de brevet français N° 2 446 001 décrit la possibilité d'utiliser des fibres de carbone pour former un conducteur. Or, l'utilisation de telles fibres de carbone serait avantageuse dans le cas d'une sonde coaxiale, telle que celle mentionnée ci-dessus. Cependant, le problème qui se pose est celui de réaliser les moyens de blocage unidirectionnels, pour une sonde dont au moins un conducteur est formé d'une pluralité de fibres. En effet, les moyens de blocage unidirectionnels décrits par l'art antérieur ne sont pas compatibles avec la réalisation d'au moins un conducteur sous forme de fibres.

Les buts de l'invention sont donc de pallier les inconvénients des sondes connues et d'apporter une solution à ce problème.

Un premier but est de fournir une sonde, notamment pour stimulateur cardiaque, dont l'accrochage des extrémités respectivement sur l'oreillette et le ventricule est réalisé correctement.

Un second but est de fournir une sonde unique comportant une connexion à l'oreillette et une connexion au ventricule, situées à une partie extrême, adaptable quelle que soit la distance entre l'oreillette et le ventricule, et dont l'installation et les manipulations sont aisées.

Pour parvenir à ces buts, l'invention propose une sonde susceptible d'être associée notamment à un stimulateur cardiaque par une de ses parties extrêmes, et par deux connexions situées à sa partie extrême opposée, respectivement à l'oreillette et au ventricule cardiaque, caractérisée par le fait que les moyens de blocage unidirectionnel du conducteur sont constitués par des moyens d'effacement des fibres constituant au moins une connexion cardiaque, de préférence une connexion cardiaque en retrait.

Suivant une autre caractéristique de l'invention, les connexions cardiaques sont écartées le long de l'axe de la sonde d'une distance qui est variable.

Une telle sonde présente donc l'avantage de posséder deux connexions cardiaques, qui grâce à la mobilité des deux conducteurs l'un par rapport à l'autre, sont disposées à une distance variable, c'est-à-dire réglable et adaptable selon les nécessités. Par coulissement d'un conducteur relativement à l'autre, on fait varier la distance entre les deux connexions et la sonde peut donc être adaptée au cœur de tout patient. La sonde selon l'invention possède également les avantages conférés par le fait qu'au moins un conducteur est formé de fibres.

Selon une autre caractéristique de l'invention, les moyens de coulissement axial des deux conducteurs sont deux tubes, ou gaines, imbriqués, mobiles l'un par rapport à l'autre grâce à un espace radial ménagé entre eux, ou jeu opposé.

Les autres caractéristiques de l'invention seront bien comprises grâce à la description qui suit, en référence aux dessins annexés dans lesquels :

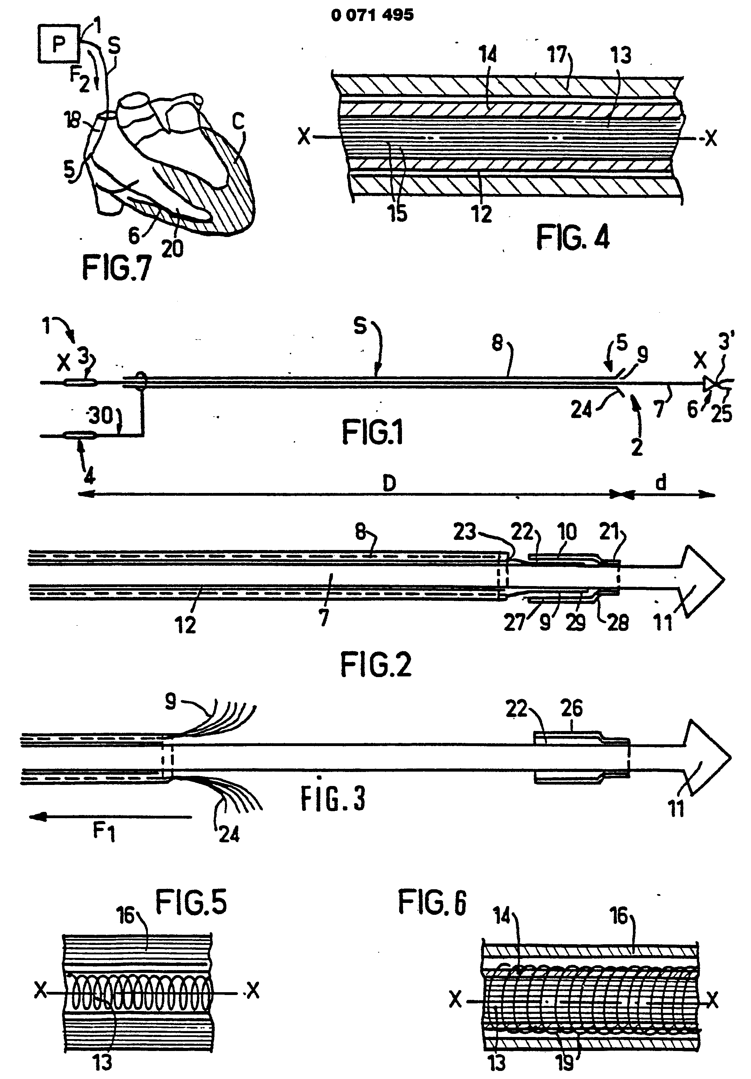

la figure 1 est une vue schématique de la sonde selon l'invention ;

la figure 2 est une vue à plus grande échelle de la partie extrême de la sonde destinée à être associée au cœur avant sa mise en place ;

la figure 3 est une vue de la partie extrême de la sonde après sa mise en place ;

la figure 4 est une coupe schématique d'une première forme de réalisation des conducteurs de la sonde selon l'invention ;

la figure 5 est une coupe d'une deuxième forme de réalisation des conducteurs ;

la figure 6 est une coupe d'une troisième forme de réalisation des conducteurs ;

la figure 7 est une vue schématique d'une sonde selon l'invention introduite dans le cœur.

Selon l'invention, il est proposé une sonde S dont la partie extrême 1 est destinée à être associée mécaniquement et électriquement, de façon connue, par deux connexions 3, 4 à un stimulateur P, et respectivement au ventricule et à l'oreillette cardiaque par deux connexions électriques et mécaniques 5, 6, l'une en retrait 5, l'autre terminale 6, et situées à sa partie extrême opposée 2.

La sonde S tendue est rectiligne, présente un axe XX, et a une forme sensiblement au moins pseudo cylindrique de révolution autour de l'axe XX. La sonde S est souple pour pouvoir être adaptée au cheminement spécifique entre le stimulateur P et le cœur C. Dans son ensemble et sous réserve de ce qui est expliqué en détail ultérieurement, elle est sensiblement inextensible.

Suivant une caractéristique de l'invention, la sonde S comporte deux conducteurs 7, 8 électriques formant un ensemble monobloc, c'est-à-dire non dissociables, associés entre eux par des moyens permettant leur coulissement axial l'un par rapport à l'autre, le long de l'axe XX. Les deux conducteurs 7, 8 correspondent respectivement à la connexion cardiaque et la connexion cardiaque terminale 6 en retrait 5.

Les connexions cardiaques 5, 6 sont écartées l'une de l'autre le long de l'axe XX de la sonde S, d'une distance d, variable lorsque les conducteurs 7, 8 sont déplacés l'un par rapport à l'autre, la connexion 6 constituant à proprement parler l'extrémité libre correspondante de la sonde S, alors que la connexion 5 est en retrait, à l'arrière, de la connexion 6.

Les connexions cardiaques 5, 6 sont situées respectivement aux extrémités libres des conducteurs 7, 8, du côté de la partie extrême 2. En faisant coulisser par exemple le conducteur 8, par rapport au conducteur 7, dans le sens de la flèche $F_1$, dirigée depuis l'extrémité 2 de la sonde vers son extrémité 1, on fait varier en l'occurrence augmenter la distance d entre les connexions cardiaques 5, 6. L'un des conducteurs, de préférence le conducteur 8, comporte du mou à sa partie extrême 30 destinée à être associée au stimulateur P, pour permettre le coulissement relatif.

Selon une autre caractéristique de l'invention, la sonde S comporte des moyens de blocage unidirectionnels 10 du conducteur 8 par rapport au conducteur 7. Les moyens de blocage 10 empêchent le coulissement du conducteur 8 par rapport au conducteur 7 dans le sens opposé à la flèche $F_1$, au-delà d'une position limite correspondant à la valeur minimale de d.

Les moyens de blocage unidirectionnels 10 sont de préférence situés au niveau de la connexion 5 en retrait. De préférence, les moyens de blocage unidirectionnels 10 sont constitués par une bague 10 coaxiale aux conducteurs 7, 8 dont une extrémité 21 est fixée rigidement au conducteur 7 et dont l'extrémité opposée 22 dirigée vers la connexion 5, vient en butée contre un décrochement annulaire 23 du conducteur 8, au voisinage immédiat de son extrémité libre.

Les connexions cardiaques 5, 6 sont de préférence constituées par une pluralité de fibres respectivement 9 conductrices d'électricité, constituant les parties terminales de fibres formant les conducteurs respectifs, pouvant être ancrées dans le muscle cardiaque, divergeant de l'axe XX de la sonde S, et formant une sorte de plumeau 24, 25, élastiquement déformable. Les fibres prennent cette forme en plumeau en l'absence d'action extérieure. Ainsi, lorsque l'on fait pénétrer les fibres dans le cœur, la zone cardiaque qui est en contact avec l'extrémité des fibres est déterminée par l'intersection de cette surface cardiaque avec l'enveloppe de fibres. De préférence, les fibres sont situées dans un cône d'axe XX dont la grande base est dirigée vers l'extrémité libre des fibres.

La surface d'intersection entre la surface cardiaque et l'enveloppe des fibres est donc supérieure à celle qui serait obtenue si les fibres étaient parallèles à l'axe XX, donc à l'intérieur d'un cylindre. L'ancrage des fibres dans le muscle cardiaque est ainsi amélioré. Les fibres 9 peuvent par exemple être des fibres de carbone.

Selon une autre caractéristique de l'invention, la sonde S comporte des moyens d'effacement 26 temporaire du plumeau 24, 25 de fibres d'au moins une connexion cardiaque 5, 6 lors de sa mise en place, de préférence la connexion 5 en retrait. Plus particulièrement, les moyens d'effacement 26 des fibres 9 sont constitués par les moyens de blocage unidirectionnels 10 du conducteur 8 par rapport au conducteur 7. Les moyens d'effacement sont donc constitués par la bague 10 enserrant le plumeau 24 de fibres. La bague 10, outre sa partie terminale 21 de fixation au conducteur 7, dirigée vers la connexion 6, et sa partie extrême 22 de butée contre le décrochement 23 du conducteur 8, présente une partie latérale 27 de maintien du plumeau 24 et une partie médiane 28 de blocage frontal des fibres 9 du plumeau 24. La partie latérale 27 et le conducteur 7 sont coaxiaux, pseudocylindriques, et définissent un espace annulaire 29 d'effacement du plumeau 24. Les moyens d'effacement 26 sont donc intégrés à demeure à la sonde et en font partie intégrante. Ce ne sont pas des moyens extérieurs. Les moyens d'effacement 26 ne sont actifs que lors de la mise en place de la sonde, les deux conducteurs 7, 8 ayant une position relative particulière, la connexion cardiaque en retrait 5 étant la plus proche de la connexion cardiaque 6, donc voisine de l'extrémité libre 11 de la sonde S, la distance d étant minimale. Lorsque l'on effectue un coulissement relatif des deux conducteurs 7, 8 l'un par rapport à l'autre, selon la flèche $F_1$, à partir de la position de mise en place de la sonde, c'est-à-dire lorsque la connexion 5 en retrait et la connexion 6 sont écartées l'une de l'autre, la distance d augmentant, les moyens d'effacement sont passifs et les fibres 9 sont dégagées de la bague 10, pour former le plumeau 24.

Selon une autre caractéristique de l'invention les moyens de coulissement axial des deux conducteurs 7, 8 sont deux tubes, ou gaines, imbriqués, mobiles l'un par rapport à l'autre, un espace ou jeu 12 radial étant ménagé entre les tubes. Cet espace permet le coulissement axial des conducteurs 7, 8.

Selon une première variante de l'invention, le conducteur interne 7 est de forme sensiblement cylindrique, d'axe XX et formé d'un fil conducteur d'électricité 13 noyé dans une gaine 14 isolante. Le fil conducteur 13 est constitué d'une pluralité de fibres 15 d'un matériau conducteur d'électricité, de préférence des fibres de carbone. La gaine isolante 14 est par exemple constituée de polyéthylène. Le fil conducteur d'électricité 13 peut être disposé dans une gaine isolante externe 14, elle aussi par exemple, en polyéthylène. Par ailleurs, on peut prévoir un fil conducteur 13 en forme de spirale (figure 5), s'enroulant hélicoïdalement autour de l'axe XX.

Selon cette première variante, le conducteur externe 8 est constitué par un fil conducteur externe 17 noyé dans une gaine externe 16 isolante, coaxiale à la gaine 14 du conducteur interne 7. Ce fil conducteur d'électricité externe 17 peut être constitué par exemple d'une pluralité de fibres conductrices d'électricité, à savoir des fibres de carbone. Entre la gaine interne 14 et la gaine externe 16, un espace ou jeu 12 est ménagé, comme on l'a mentionné plus haut, pour permettre le coulissement des deux conducteurs 7, 8.

Selon une deuxième variante, le conducteur externe 8 est constitué par un fil conducteur spiralé 19 s'enroulant hélicoïdalement autour de la gaine isolante 14 et compris entre celle-ci et une gaine externe coaxiale 16. Entre les spires du fil spiralé 19 et la gaine isolante 14, il existe un espace annulaire 12 ou jeu qui permet le coulissement du conducteur 8 par rapport au conducteur 7. Ainsi, les spires du fil 19 ne serrent pas la gaine 14. Les gaines internes et externes sont mobiles l'une par rapport à l'autre, librement ou à frottement doux grâce au jeu 12 qui est ménagé entre elles.

Pour mettre en place la sonde S selon l'invention, on procède de la manière suivante : on introduit selon la flèche $F_2$, dirigée en sens opposé à la flèche $F_1$, l'extrémité libre 11 de la sonde S dans une veine, de préférence située à la base du cou. Les moyens d'effacement de préférence du plumeau 24 sont alors actifs et les fibres 9 sont effacées. Les deux conducteurs 7 et 8 ne sont pas mobiles l'un par rapport à l'autre, les moyens de blocage unidirectionnels 26 du conducteur 8 par rapport au conducteur 7 étant actifs, la partie extrême 22 de ces moyens venant en butée contre le décrochement 23 du conducteur 8, comme expliqué ci-dessus. L'extrémité libre 11 de la sonde S est introduite jusque dans le ventricule 20, en passant par l'oreillette 18. On libère alors la connexion cardiaque 6. Celle-ci est fixée dans le ventricule 20. Ainsi, lors de l'introduction de la sonde S dans la veine et jusqu'à la fixation de la connexion cardiaque 6 dans le ventricule 20, les deux conducteurs respectivement interne et externe 7, 8 ont été déplacés simultanément et il n'y a pas eu de coulissement relatif de l'un par rapport à l'autre. Après fixation de la connexion cardiaque 6, on effectue un déplacement axial du conducteur externe 8 dans le sens de la flèche $F_1$. Les moyens d'effacement 26 des fibres 9 sont alors inactifs et celles-ci sont libérées. Comme elles sont élastiquement déformables, elles s'écartent naturellement de l'axe XX pour prendre une forme de plumeau 24. Enfin, au moment où les fibres sont au niveau du point optimal de recueil auriculaire, elles vont être fixées dans l'oreillette 18.

Ainsi, la distance d entre le point de recueil auriculaire et le point de recueil ventriculaire variant selon les patients, il suffit pour adapter la sonde, de faire plus ou moins coulisser le conducteur externe 8 par rapport au conducteur interne 7 dans le sens de la flèche $F_1$ dirigée de la partie extrême 2 de la sonde associé au muscle cardiaque vers la partie extrême 1.

**Revendications**

1. Sonde destinée à être associée par une partie extrême notamment à un stimulateur cardiaque et par deux connexions cardiaques (5, 6) disposées à la partie extrême opposée (2), respectivement à l'oreillette (18) et au ventricule cardiaque (20), comportant deux conducteurs (7, 8) formant un ensemble monobloc et associés entre eux par des moyens permettant leur coulissement axial l'un par rapport à l'autre, au moins un conducteur étant formé d'une pluralité de fibres (15) conductrices d'électricité, notamment de carbone, comportant des moyens de blocage unidirectionnels (10) d'un conducteur (8) par rapport à l'autre conducteur (7), caractérisée par le fait que les moyens de blocage unidirectionnel (10) du conducteur (8) sont constitués par des moyens d'effacement (26) des fibres constituant au moins une connexion cardiaque, de préférence une connexion cardiaque en retrait (5).

2. Sonde selon la revendication 1, caractérisée par le fait que les moyens d'effacement (26) sont constitués par une bague (10) faisant partie intégrante de la sonde, comportant une partie terminale (21) de fixation au conducteur (7), une partie latérale (27) de maintien de fibres (9) et une partie médiane (28) de blocage frontal des fibres (9), la partie latérale (27) et le conducteur (7) définissant un espace annulaire (29) d'effacement.

3. Sonde selon les revendications 1 et 2, caractérisée par le fait que les moyens de blocage unidirectionnels du conducteur (8) par rapport au conducteur (7) sont constitués par la partie extrême (22) de la bague (10) venant en butée contre un décrochement annulaire (23) du conducteur (8).

4. Sonde selon l'une quelconque des revendications 1 à 3, caractérisée en ce que les moyens de coulissement axial sont constitués par deux tubes ou gaines (14, 16) imbriqués, mobiles l'un par rapport à l'autre, un espace annulaire (12) étant ménagé entre les tubes (14, 16).

5. Sonde selon la revendication 4, caractérisée en ce que les tubes (14, 16) sont de forme sensiblement cylindrique, coaxiaux, leur axe étant constitué par l'axe XX de la sonde S.

6. Sonde selon l'une quelconque des revendications 1 à 5, caractérisée en ce qu'au moins un conducteur (7, 8) est constitué par un fil conducteur (13, 19) de forme sensiblement hélicoïdale, s'entourant autour de l'axe XX.

7. Sonde selon l'une quelconque des revendications 1 à 6, caractérisée en ce qu'au moins un conducteur (7, 8) est noyé dans une gaine isolante (14).

8. Sonde selon l'une quelconque des revendications 1 à 8, caractérisée en ce que les connexions cardiaques (5, 6) sont écartées l'une de l'autre le long de l'axe XX de la sonde S, la sonde cardiaque (6) étant située à l'extrémité libre de la sonde (11) et la connexion cardiaque (5) étant située en retrait.

## Claims

1. A probe designed to be connected by one end to a heart pace-maker in particular, and to the auricle (18) and the ventricle (20) via two cardiac connections (5, 4) at the opposite end (2), comprising two conductors (7, 8) forming a monobloc unit and connected to one another by means which allow them to slide axially with respect to one another, at least one conductor being formed by a plurality of fibres (15) which are electrically conductive, in particular made of carbon, comprising unidirectional means for blocking the conductor with respect to the other conductor (7), characterised in that the unidirectional blocking means (10) for the conductor (8) comprises fibre-isolating means (26) forming at least one cardiac connexion, preferably a recessed cardiac connexion (5).

2. A probe according to claim 1, characterised in that the blocking means (26) comprises a ring (10) forming an integral part of the probe, comprising a terminal portion (21) for securing to the conductor (7), a lateral portion (27) for supporting the fibres (9) and a central portion (28) for frontal blockage of the fibres (9), the lateral portion (27) and the conductor (7) defining an annular blocking space (29).

3. A probe according to claims 1 and 2, characterised in that the unidirectional means (10) for blocking the conductor (8) with respect to the conductor (7) comprise the end portion (22) of the ring (10) abutting against an annular unhooking device (23) of the conductor (8).

4. A probe according to any one of claims 1 to 3, characterised in that the axial sliding means are comprised of two overlapping tubes or sleeves (14, 16), which are movable with respect to one another, an annular space (12) being left between the tubes (14, 16).

5. A probe according to claim 4, characterised in that the tubes (14, 16) are approximately cylindrical in shape and are coaxial, their axis being formed by the axis XX of the probe S.

6. A probe according to any one of claims 1 to 5, characterised in that at least one conductor (7, 8) is formed by a conductive wire (13, 19), approximately helicoidal in shape, wound round the axis XX.

7. A probe according to any one of claims 1 to 6, characterised in that at least one conductor (7, 8) is embedded in an insulating sleeve (14).

8. A probe according to any one of claims 1 to 7, characterised in that the cardiac connexions (5, 6) are separated from one another along the axis XX of the probe S, the cardiac probe being situated at the free end of the probe (11) and the cardiac connexion (5) being recessed.

## Patentansprüche

1. Sonde, die mittels ihres einen Endbereichs insbesondere mit einem Herzschrittmacher und mittels zweier Herzanschlüsse (5, 6), die am gegenüberliegenden Endbereich angeordnet sind, mit einem Herzvorhof (18) bzw. einer Herzkammer (20) verbindbar ist, mit zwei Leitern (7, 8), die eine Einheit bilden und die miteinander durch ein Mittel verbunden sind, das deren axiale Verschiebung zueinander zulässt, wobei wenigstens ein Leiter durch eine Vielzahl elektrisch leitender Fasern (15), insbesondere aus Kohlenstoff gebildet ist, und mit einem Mittel (10) zum Verriegeln eines Leiters (8) gegenüber dem anderen Leiter (7) in einer Richtung, dadurch gekennzeichnet, daß das in einer Richtung wirkende Verriegelungsmittel (10) des Leiters (8) durch ein Mittel (26) zum Rückstellen der Fasern, die zumindest einen Herzanschluß, vorzugsweise einen zurückspringenden Herzanschluß (5) bilden, gebildet ist.

2. Sonde nach Anspruch 1, dadurch gekennzeichnet, daß das Rückstellmittel (26) durch einen Ring (10) gebildet ist, der integraler Bestandteil der Sonde ist und der einen Endbereich (21) zum Befestigen am Leiter (7), einen Längsbereich (27) zum Halten der Fasern (9) und einen Mittenbereich (28) zum stirnseitigen Anhalten der Fasern (9) aufweist, wobei der Längsbereich (27) und der Leiter (7) einen ringförmigen Rückstellraum (29) bilden.

3. Sonde nach den Ansprüchen 1 und 2, dadurch gekennzeichnet, daß das in einer Richtung wirkende Verriegelungsmittel des Leiters (8) im Verhältnis zum Leiter (7) durch ein Endteil (22) des Ringes (10) gebildet ist, das als Widerlager für einen ringförmigen Teil (23) des Leiters (8) dient.

4. Sonde nach einem der Ansprüche 1 bis 3, dadurch gekennzeichnet, daß das axiale Verschiebemittel durch zwei ineinander greifende Rohre oder Hülsen (14, 16) gebildet sind, die zueinander beweglich sind, wobei ein ringförmiger Raum (12) zwischen den Rohren (14, 15) vorhanden ist.

5. Sonde nach Anspruch 4, dadurch gekennzeichnet, daß die Rohre (14, 16) etwa zylindrisch und koaxial sind, wobei ihre Achse durch die Achse (XX) der Sonde (S) gebildet ist.

6. Sonde nach einem der Ansprüche 1 bis 5, dadurch gekennzeichnet, daß wenigstens ein Leiter (7, 8) durch einen im wesentlichen wendelförmigen Leiterdraht (13, 19) gebildet ist, der die Achse (XX) umgibt.

7. Sonde nach einem der Ansprüche 1 bis 6, dadurch gekennzeichnet, daß wenigstens ein Leiter (7, 8) in einer Isolierhülle (14) eingebettet ist.

8. Sonde nach einem der Ansprüche 1 bis 8, dadurch gekennzeichnet, daß die Herzanschlüsse (5, 6) längs der Achse (XX) der Sonde (S) voneinander entfernt sind, wobei die Herzsonde (6) am freien Ende der Sonde (11) und der Herzanschluß (5) zurückgezogen angeordnet ist.

FIG.7

FIG. 4

FIG.1

FIG.2

FIG.3

FIG.5

FIG.6